# EUROPEAN PATENT APPLICATION

(11) **EP 4 570 971 A1**
(43) Date of publication of application: **18.06.2025**
(21) Application number: 23846314.5
(22) Date of filing: 19.07.2023
(51) Int. Cl.: C40B 50/06, C12N 15/11, C12Q 1/6869

(54) **FRAGMENTATION AND TAGMENTATION METHOD FOR NEXT-GENERATION SEQUENCE ANALYSIS AND LIKE**

(30) Priority: 25.07.2022 JP 2022118079
(71) Applicant: Toyobo Co., Ltd., Kita-ku Osaka-shi, Osaka 530-0001 (JP)
(72) Inventor: TAKEDA, Taka-aki, Tsuruga-shi, Fukui 914-8550 (JP); KOBAYASHI, Tetsuhiro, Tsuruga-shi, Fukui 914-8550 (JP)
(74) Representative: Vossius & Partner Patentanwälte Rechtsanwälte mbB
(86) International application number: PCT/JP2023/026339
(87) International publication number: WO 2024/024583

(57) **Abstract**

Provided is a method for efficiently generating a library of double-stranded DNA having different tagment sequences on the 5' end and 3' end sides in a simple procedure. The method comprises the following steps (i) to (iii):
(i) fragmenting double-stranded DNA using a transposome comprising (a) a first adapter containing a first tagment sequence and a first transposon end sequence, (b) a second adapter containing a second transposon end sequence having a base sequence complementary to at least a portion of the first transposon end sequence, and (c) a transposase, and linking either the first adapter or the second adapter as a transferred strand to each of the 5' ends or 3' ends of the fragmented double-stranded DNA;
(ii) linking an additional oligo containing a second tagment sequence to the second adapter; and
(iii) linking the first adapter or the second adapter that is not linked to the fragmented double-stranded DNA, as a non-transferred strand, to each of the 5' ends or 3' ends of the fragmented double-stranded DNA to which the adapter as the transferred strand is not linked in step (i).

## Description

### Technical Field

The present invention relates to a method for generating a double-stranded DNA library and the like.

### Background Art

In recent years, analysis using next-generation sequencers (NGS) has been widely used, and various pretreatment techniques for this analysis, such as fragmentation and tagmentation of double-stranded DNA, have been developed. For example, in a known method, a first tagment sequence or a second tagment sequence is provided to a recombinant enzyme, such as a transposase, to form two types of transposomes, and target double-stranded DNA is randomly fragmented and tagmented by the two types of transposomes. However, this method is problematic in that the two types of transposomes randomly bind to the ends of the target double-stranded DNA, which reduces the theoretical yield by one-half, strand information cannot be retained, and it cannot be used for bisulfite sequencing analysis.

In RNA sequencing analysis etc., emphasis has been recently placed on which strand of double-stranded genomic DNA the RNA is transcribed from, i.e., RNA sequencing analysis methods that retain strand information. The use of strand information enables not only analysis of non-coding genes such as antisense RNA, but also more accurate quantification of expression of overlapping genes. Thus, the development of methods for fragmentation and tagmentation to retain strand information has progressed. For example, a method comprising adding Y-adapters after fragmentation (Patent Literature (PTL) 1) and methods comprising performing fragmentation using a transposase, tagmenting only the 5' side with a first tagment sequence, removing the adapter at the 3' end by heat treatment or the like, and performing replacement with a second tagment sequence (PTL 2 and Non-patent Literature (NPL) 1) have been reported so far.

### Citation List

### Patent Literature

PTL 1: US Patent No. 8563478
PTL 2: US Patent No. 10287574

### Non-patent Literature

NPL 1: Wang et al., Nature Protocols Vol. 8, No. 10, 2022-2032, 2013

### Summary of Invention

### Technical Problem

Conventional methods of performing fragmentation and tagmentation of double-stranded DNA while retaining strand information often involve a complicated procedure or low collection efficiency, or both. For example, in PTL 1, fragmentation reaction and adenine addition reaction are usually required before adding Y-adapters, and further, it is necessary to remove adapter dimers after addition of the Y-adapters, resulting in a complicated procedure and low collection efficiency. In PTL 2 and NPL 1, as is clear from the Examples, a procedure in which the adapter at the 3' end is removed, and a new adapter containing another tagment sequence is provided, is required after fragmentation by a transposase and tagmentation only at the 5' end, and temperature changes in multiple steps are required to complete this procedure. Such a multistep process is a complicated procedure and causes losses at each step of the procedure, resulting in a reduced yield of the ultimately generated double-stranded DNA library. An object of the present invention is to provide a method for efficiently fragmenting and tagmenting double-stranded DNA in a simple procedure.

### Solution to Problem

The present inventors conducted extensive research to solve the above problems and found a method for efficiently fragmenting double-stranded DNA and adding different tagment sequences to the 5' end and 3' end sides in a procedure that is simpler than conventional methods. Specifically, the inventors found that fragmentation and addition of first and second tagment sequences can be performed in a simple and efficient manner by performing fragmentation of double-stranded DNA with a transposase to which the first tagment sequence is provided, and then linking, without removing the adapter on the 3' end side, the second tagment sequence to the adapter with a ligase or the like. The present invention has been accomplished by conducting further research based on this finding.

Specifically, the present invention typically encompasses the following embodiments.

### [Item 1]

A method for generating a library of double-stranded DNA having different tagment sequences on the 5' end and 3' end sides, the method comprising the following steps (i) to (iii):
(i) fragmenting double-stranded DNA using a transposome comprising (a) a first adapter containing a first tagment sequence and a first transposon end sequence, (b) a second adapter containing a second transposon end sequence having a base sequence complementary to at least a portion of the first transposon end sequence, and (c) a transposase, and linking either the first adapter or the second adapter as a transferred strand to each of the 5' ends or 3' ends of the fragmented double-stranded DNA;
(ii) linking an additional oligo containing a second tagment sequence to the second adapter; and
(iii) linking the first adapter or the second adapter that is not linked to the fragmented double-stranded DNA, as a non-transferred strand, to each of the 5' ends or 3' ends of the fragmented double-stranded DNA to which the adapter as the transferred strand is not linked in step (i),
step (ii) being performed before, after, or simultaneously with step (iii).

### [Item 2]

The method according to Item 1, wherein the first adapter contains the first tagment sequence at a position closer to the 5' end than the first transposon end sequence, and serves as a transferred strand that is linked to each of the 5' ends of the fragmented double-stranded DNA using the transposase.

### [Item 3]

The method according to Item 1 or 2, wherein the additional oligo contains a base sequence complementary to a portion of the base sequence of the first adapter.

### [Item 4]

The method according to any one of Items 1 to 3, wherein the transposase is hyperactive Tn5 transposase, and the first and second transposon end sequences are Tn5-type transposon end sequences.

### [Item 5]

The method according to any one of Items 1 to 4, wherein in step (ii), the additional oligo is linked to the second adapter using a DNA ligase.

### [Item 6]

The method according to any one of Items 1 to 5, wherein in step (iii), the adapter as the non-transferred strand that is not linked to the fragmented double-stranded DNA is linked to the fragmented double-stranded DNA using a DNA polymerase and a DNA ligase.

### [Item 7]

The method according to any one of Items 1 to 6, wherein in step (iii), the adapter as the non-transferred strand that is not linked to the fragmented double-stranded DNA is the second adapter whose 5' end is modified by phosphorylation, and the second adapter is linked to each of the 3' ends of the fragmented double-stranded DNA.

### [Item 8]

The method according to any one of Items 1 to 7, wherein in step (i), the first adapter is linked as the transferred strand to each of the 5' ends of the fragmented double-stranded DNA, and in step (ii), the additional oligo whose 5' end is modified by phosphorylation is linked to the 3' end of the second adapter.

### [Item 9]

The method according to any one of Items 1 to 8, wherein in step (i), the first adapter is linked as the transferred strand to each of the 5' ends of the fragmented double-stranded DNA, and in step (ii), the additional oligo in which the OH group on the 3' end side is modified is linked to the 3' end of the second adapter.

### [Item 10]

The method according to Item 9, wherein the modification of the OH group is a modification with a phosphate group or inverted dT.

### [Item 11]

The method according to any one of Items 1 to 10, wherein a double-stranded DNA library that retains strand information is generated.

### [Item 12]

The method according to any one of Items 1 to 11, wherein at least one strand of the fragmented double-stranded DNA is modified.

### [Item 13]

The method according to Item 12, wherein the modification is a methylation modification.

### [Item 14]

The method according to Item 12, wherein a double-stranded DNA library for use in bisulfite analysis is generated using the first adapter in which a cytosine base in the first tagment sequence is methylated, and the additional oligo in which a cytosine base in the second tagment sequence is methylated.

### Advantageous Effects of Invention

The present invention makes it possible to efficiently fragment double-stranded DNA and add different tagment sequences to the 5' end and 3' end sides in a procedure that is simpler than conventional methods. The method of the present invention eliminates the need for preparing two different types of transposomes. The method of the present invention also enables strand information to be easily retained. This allows for efficient and advanced sequencing analysis, including NGS, from an extremely small amount of a sample, greatly contributing to advancing gene analysis and diagnostic techniques.

### Brief Description of Drawings

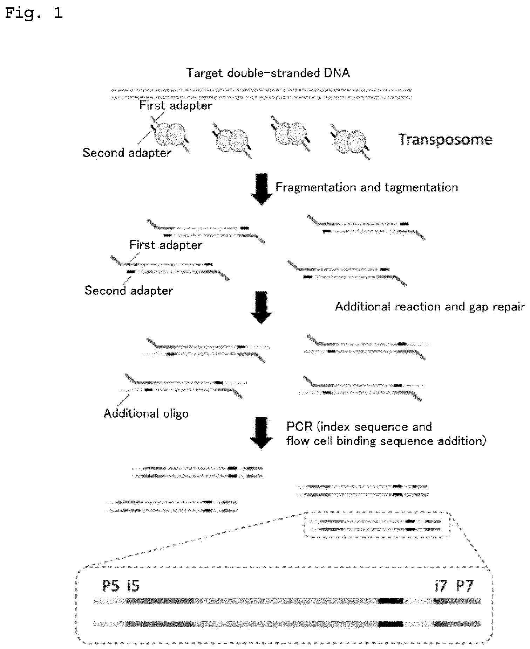

Fig. 1 schematically shows an embodiment of the present invention. After a first adapter having a first tagment sequence and a first transposon end sequence, and a second adapter having a second transposon end sequence are added to double-stranded DNA by using a tagmentase, such as a transposase, an additional oligo having a second tagment sequence is linked to the second adapter, and the adapter (non-transferred strand; the second adapter in Fig. 1) that is not linked (covalently bound) to the double-stranded DNA is linked to the double-stranded DNA, thereby enabling generation of a library of double-stranded DNA having different tagment sequences on the 5' end and 3' end sides.

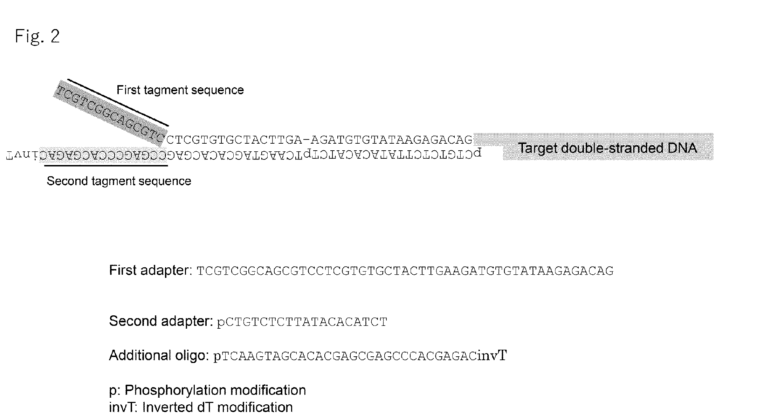

Fig. 2 is a schematic diagram of an embodiment of the present invention in which a first adapter consisting of the base sequence set forth in SEQ ID NO: 3, a second adapter consisting of the base sequence set forth in SEQ ID NO: 4, and an additional oligo consisting of the base sequence set forth in SEQ ID NO: 5 are used. The 5' ends of the second adapter and the additional oligo are modified by phosphorylation to allow linking to adjacent DNA. The 3' end of the additional oligo is modified with inverted dT to prevent degradation of the oligo and to prevent it from functioning as a primer in PCR reaction etc.

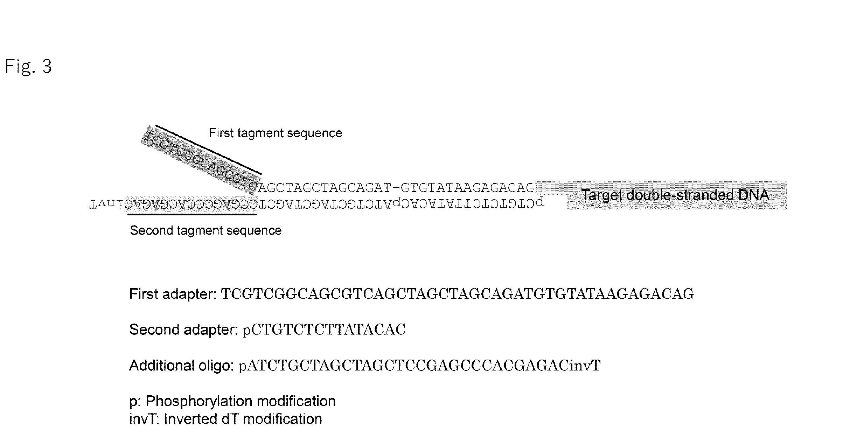

Fig. 3 is a schematic diagram of an embodiment of the present invention in which a first adapter consisting of the base sequence set forth in SEQ ID NO: 6, a second adapter consisting of the base sequence set forth in SEQ ID NO: 7, and an additional oligo consisting of the base sequence set forth in SEQ ID NO: 8 are used (an example in which the length of each of the base sequences of the first adapter and the second adapter is shorter than that in the example shown in Fig. 2). The 5' ends of the second adapter and the additional oligo are modified by phosphorylation to allow linking to adjacent DNA. The 3' end of the additional oligo is modified with inverted dT to prevent degradation of the oligo and to prevent it from functioning as a primer in PCR reaction etc.

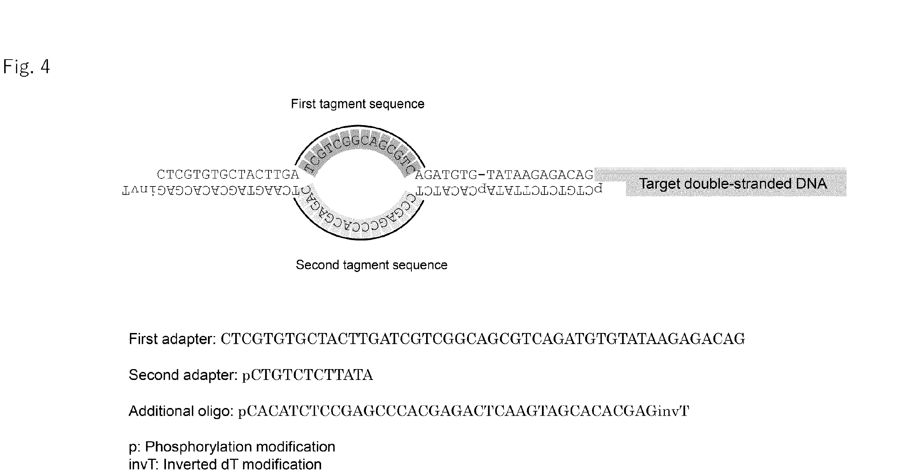

Fig. 4 is a schematic diagram of an embodiment of the present invention in which a first adapter consisting of the base sequence set forth in SEQ ID NO: 9, a second adapter consisting of the base sequence set forth in SEQ ID NO: 10, and an additional oligo consisting of the base sequence set forth in SEQ ID NO: 11 are used (an example in which the first tagment sequence in the first adapter and the second tagment sequence in the additional oligo are not located at the ends, and the first and second tagment sequences form a cyclic structure). The 5' ends of the second adapter and the additional oligo are modified by phosphorylation to allow linking to adjacent DNA. The 3' end of the additional oligo is modified with inverted dT to prevent degradation and to prevent it from functioning as a primer in PCR reaction etc.

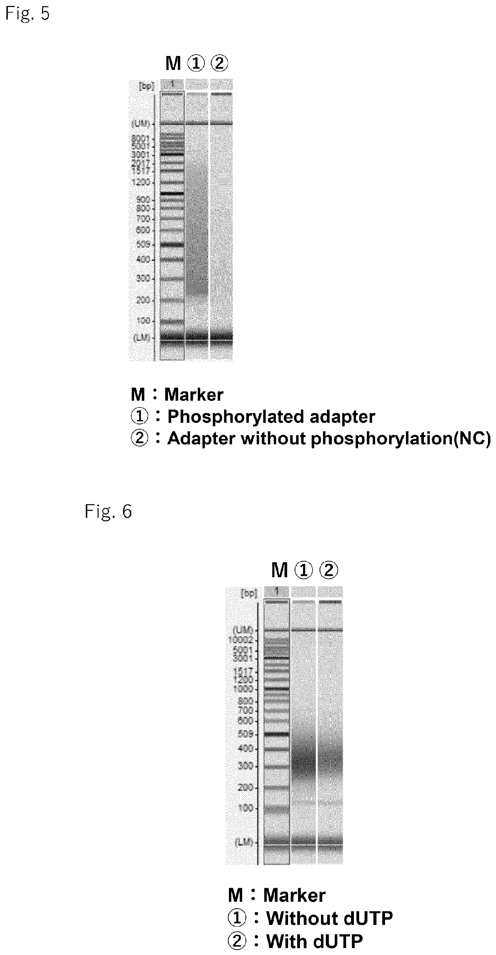

Fig. 5 shows the results of electrophoresis of library products (adapters 2) in Example 1. The patterns of the library products were confirmed using a Microchip Electrophoresis System for DNA/RNA Analysis MultiNA (Shimadzu Corporation).
Fig. 6 shows the results of electrophoresis of library products (adapters 3) in Example 2. The patterns of the library products were confirmed using a Microchip Electrophoresis System for DNA/RNA Analysis MultiNA (Shimadzu Corporation).

### Description of Embodiments

### Disclosure of Invention

The present invention is described in more detail below while describing embodiments of the present invention.

### Method for Generating Double-stranded DNA Library

The present invention provides a method for generating a library of double-stranded DNA having different tagment sequences on the 5' end and 3' end sides in a procedure that is simpler than conventional methods. In the present invention, double-stranded DNA is fragmented using a transposome comprising a first adapter containing a first tagment sequence, a second adapter, and a transposase, and then an additional oligo containing a second tagment sequence is linked to the second adapter using a ligase or the like, thereby enabling simple and efficient fragmentation of double-stranded DNA and addition of the first tagment sequence and the second tagment sequence. The method of the present invention enables fragmentation and addition of tagment sequences, which are necessary for sequencing analysis, including NGS. In the tagmentation of the present invention, the different tagment sequences are added to the 5' end side and the 3' end side, and thus, for example, a double-stranded DNA library can be prepared with strand information retained.

### 1. Addition of First Tagment Sequence and Second Tagment Sequence

In the present invention, a library of double-stranded DNA having different tagment sequences on the 5' end and 3'end sides is generated by a method comprising at least the following steps (i) to (iii):
(i) fragmenting double-stranded DNA using a transposome comprising (a) a first adapter containing a first tagment sequence and a first transposon end sequence, (b) a second adapter containing a second transposon end sequence having a base sequence complementary to at least a portion of the first transposon end sequence, and (c) a transposase, and linking either the first adapter or the second adapter as a transferred strand to each of the 5' ends or 3' ends of the fragmented double-stranded DNA;
(ii) linking an additional oligo containing a second tagment sequence to the second adapter; and
(iii) linking the first adapter or the second adapter that is not linked to the fragmented double-stranded DNA, as a non-transferred strand, to each of the 5' ends or 3' ends of the fragmented double-stranded DNA to which the adapter as the transferred strand is not linked in step (i).

The order of steps (ii) and (iii) among these steps is not limited. Step (ii) may be performed before step (iii), step (iii) may be performed before step (ii), or step (ii) and step (iii) may be performed simultaneously.

In the present invention, in step (i), double-stranded DNA is fragmented using a transposome comprising components (a), (b), and (c) described above, and the first adapter or the second adapter is linked as the transferred strand to each of the 5' ends or the 3' ends of the fragmented double-stranded DNA. In this step, the ends to which the transferred strand is linked may be either the 5' ends or the 3' ends. In an embodiment, it is preferred that the first adapter or the second adapter is linked as the transferred strand to each of the 5' ends, and it is preferred that the first adapter is linked as the transferred strand to each of the 5' ends.

### 2. (a) First Adapter and (b) Second Adapter

In the present invention, a transposome comprising (a) a first adapter and (b) a second adapter together with (c) a transposase is used. The first adapter (a) constituting the transposome of the present invention is a polynucleotide containing at least a first tagment sequence and a first transposon end sequence. The first adapter may contain one or more base sequences of any length (for example, about 5 to 30 bp long, preferably about 7 to 25 bp long, more preferably about 15 to 20 bp long) (also referred to as "the optional sequence of the first adapter" in the present specification) in addition to the first tagment sequence and the first transposon end sequence. The optional sequence of the first adapter may be present between the first tagment sequence and the first transposon end sequence and/or may be adjacent to the first tagment sequence and on the side opposite to the first transposon end sequence (e.g., on the 5' end side of the first tagment sequence when the first adapter is linked as the transferred strand to each of the 5' ends). In a specific embodiment, the optional sequence of the first adapter is designed to complementarily bind to a portion (other than the second tagment sequence) of the additional oligo described later (preferably, the optional sequence of the first adapter is designed to complementarily bind to a portion (other than the second tagment sequence) of the additional oligo described later, together with a portion of the first transposon end sequence), and functions to facilitate linking of the additional oligo and the second adapter. In this case, the optional sequence of the first adapter is also referred to as the "sequence complementary to the additional oligo." The first tagment sequence region and the first transposon end sequence region (and the region of the optional sequence of the first adapter contained as necessary) do not necessarily have to be clearly separated, and the sequences may partially overlap.

The second adapter (b) constituting the transposome of the present invention is a polynucleotide containing at least a second transposon end sequence, preferably consisting of a second transposon end sequence. The second transposon end sequence preferably has a base sequence complementary to at least a portion of the first transposon end sequence. When the first transposon end sequence in the first adapter and the second transposon end sequence in the second adapter comprise base sequences complementary to each other as described above, the first adapter and the second adapter can form a double strand at least in a region (preferably, the region on the end side to be ultimately transferred to fragmented double-stranded DNA) via the complementary base sequences to facilitate addition to ends of fragmented double-stranded DNA.

The first tagment sequence contained in the first adapter and the second tagment sequence to be linked to the second adapter are not particularly limited, and it is preferred that the two tagment sequences, i.e., the first and second tagment sequences, are not complementary to each other. For example, the identity between a base sequence that is the reverse complement of the first tagment sequence and the base sequence of the second tagment sequence is preferably 70% or less, more preferably 60% or less, and even more preferably 50% or less. The lower limit of the identity between a base sequence that is the reverse complement of the first tagment sequence and the base sequence of the second tagment sequence is not particularly limited, and is preferably more than 0%, or preferably 1% or more. The sequence identity can be determined using a program such as BLAST or ClustalW. By designing the base sequence identity between a base sequence that is the reverse complement of the first tagment sequence and the base sequence of the second tagment sequence to be low, the first and second tagment sequences added to both ends of double-stranded DNA can be more clearly distinguished, and the 5' end and 3' end sides of double-stranded DNA to which the first or second tagment sequence is provided can be easily distinguished.

In a preferred embodiment, the first adapter (a) (in particular, a region other than the first tagment sequence, preferably the region of the optional sequence of the first adapter and/or the region of a portion of the first transposon end sequence) preferably has a base sequence of several contiguous bases (e.g., 7 bp or more, preferably 10 bp or more, more preferably 12 bp or more, even more preferably 15 bp or more; the upper limit is not particularly limited, and is, for example, 30 bp or less) that is complementary to the base sequence of the additional oligo (in particular, other than the second tagment sequence). When the base sequence of the first adapter and the base sequence of the additional oligo contain base sequences complementary to each other as described above, the additional oligo is not linked to an end of the first adapter, but easily complementarily binds to the first adapter in step (ii). This allows the additional oligo to be arranged in close proximity to the second adapter, which complementarily binds to the first adapter; as a result, the additional oligo is easily linked to an end of the second adapter.

In another specific embodiment, of the first adapter and the second adapter, the 5' end of the adapter that is not the transferred strand in step (i) is preferably modified by phosphorylation or the like. By modifying the 5' end of the adapter that is not the transferred strand in step (i) (also referred to as "the adapter as a non-transferred strand") by phosphorylation or the like, the adapter as a non-transferred strand can be easily linked to the fragmented double-stranded DNA using, for example, a DNA ligase in step (iii).

In another preferred embodiment, it is preferred that the first adapter contains the first tagment sequence at a position closer to the 5' end than the first transposon end sequence, and serves as a transferred strand that is linked to each 5' end of the fragmented double-stranded DNA using a transposase. In this embodiment, it is preferred that the second adapter is modified at the 5' end by phosphorylation or the like and serves as a non-transferred strand that can be linked to each 3' end of the fragmented double-stranded DNA using a DNA ligase or the like.

### 3. Additional Oligo

In the present invention, the oligo DNA (also referred to as "additional oligo") to be linked to the second adapter contains a second tagment sequence. The additional oligo preferably has a base sequence of several contiguous bases (e.g., 7 bp or more, preferably 10 bp or more, more preferably 12 bp or more, even more preferably 15 bp or more; the upper limit is not particularly limited, and is, for example, 30 bp or less) that is complementary to the base sequence of the first adapter (in particular, a region other than the first tagment sequence, preferably the region of the optional sequence of the first adapter and/or the region of a portion of the first transposon end sequence). The region of the second tagment sequence and the region of the base sequence complementary to the base sequence of the first adapter (in particular, a region other than the first tagment sequence, preferably the region of the optional sequence of the first adapter and/or the region of a portion of the first transposon end sequence) do not necessarily have to be clearly separated, and the sequences may partially overlap. When the additional oligo contains a base sequence complementary to the base sequence of the first adapter (in particular, a region other than the first tagment sequence, preferably the region of the optional sequence of the first adapter and/or the region of a portion of the first transposon end sequence), the additional oligo is not linked to an end of the first adapter, but easily complementarily binds to the first adapter in step (ii). This allows the additional oligo to be arranged in close proximity to the second adapter, which complementarily binds to the first adapter; as a result, the additional oligo is easily linked to an end of the second adapter.

In an embodiment, the additional oligo may contain a nucleic acid analog, such as a locked nucleic acid (LNA) or a bridged nucleic acid (BNA). Containing a nucleic acid analog, such as LNA or BNA, in the bases of the additional oligo has the advantage that the annealing temperature of the first adapter and the additional oligo can be increased, thereby facilitating complementary binding of the additional oligo to the base sequence of the first adapter.

The additional oligo used in the present invention may further contain one or more base sequences of any length (for example, about 5 to 30 bp long, preferably about 10 to 25 bp long, and more preferably about 15 to 20 bp long) (also referred to as, for example, "the optional sequence of the additional oligo" in the present specification) in addition to the second tagment sequence and the base sequence complementary to the base sequence of the first adapter. The optional sequence of the additional oligo may be present on either the 5' end or 3' end side of the second tagment sequence. From the viewpoint of lower likelihood of interfering with linking of the additional oligo to the second adapter, it is preferred that the optional sequence of the additional oligo is adjacent to the second tagment sequence and on the side opposite to the end to which the second adapter is linked (e.g., on the 3' end side of the second tagment sequence when the first adapter is linked as the transferred strand to each 5' end).

### 4. (c) Transposase and Transposon End Sequence

The transposase used in the present invention is not particularly limited, and any transposase or mutant thereof known in the art can be used. Examples include, but are not limited to, Tn3 transposase, Tn5 transposase, Tn7 transposase, Tn10 transposase, MuA transposase, Sleeping Beauty transposase, piggyBac transposase, Himar1 transposase, Mos1 transposase, and mutants thereof (e.g., mutants comprising amino acid sequences having substitution, deletion, insertion, or addition of one or several (e.g., about 1 to 10, preferably about 1 to 5, more preferably about 1 to 3) amino acids in these wild-type amino acid sequences). Among these, the following are preferable: Tn5 transposase, MuA transposase, Sleeping Beauty transposase, piggyBac transposase, Himar1 transposase, Mos1 transposase, all of which are known to have hyperactive mutants, or mutants thereof (e.g., Tn5 transposase mutants described in Picelli et al., Genome Res. 24(12): 2033-40, 2014; M. Zhou and W.S. Reznikoff, J. Mol. Biol., 271, 362-373, 1997; T.W. Wiegand and W.S. Reznikoff, J. Bacteriol., 174, 1229-1239, 1992; M.D. Weinreich, A. Gasch, and W.S. Reznikoff, Genes Dev., 8, 2363-2374, 1994; T.A. Naumann and W.S. Reznikoff, Proc. Natl. Acad. Sci. U. S. A. 97, 8944-8949, 2000; and I.Y. Goryshin and W.S. Reznikoff, J. Biol. Chem. 273, 7367-7374, 1998; Sleeping Beauty transposase mutants described in F. Voigt et al., Nat. Commun., Vol. 7, No. 11126, 2016; piggyBac transposase mutants described in K. Yusa et al., Proc. Natl. Acad. Sci. U.S.A., Vol. 108 No. 4 1531-1536, 2010; Himar1 transposase mutants described in D.J. Lampe et al., Proc. Natl. Acad. Sci. U.S.A., Vol. 96, No. 20, 11428-11433, 1999; Mos1 transposase mutants described in S. Germon et al., Genetica, Vol. 137, No. 265, 265-276, 2009; and MuA transposase mutants described in T.S. Rasila et al., Nucleic Acids Res. Vol.46, Issue 9, 4649-4661, 2018). Tn5 transposase or a mutant thereof, i.e., a Tn5-type transposase, is more preferable, and a Tn5 transposase mutant (hyperactive Tn5 transposase) is particularly preferable.

Depending on the transposase used, the transposon end sequence (a unique double-stranded sequence recognized by the transposase; usually, one strand is a transferred strand, and the other strand is a non-transferred strand) used in combination with the transposase varies. Those skilled in the art would be able to suitably select an appropriate transposon end sequence according to the type of transposase used, in accordance with common technical knowledge.

The transposon end sequence used in the present invention may not be a full-length sequence and may be a portion of a base sequence known as a transposon end sequence as long as the effects of the present invention are achieved. For example, it has been confirmed that the transposon end sequence used as a non-transferred strand can be used in the present invention if it has a length of at least 10 bp (preferably at least 11 bp, more preferably at least 12 bp). For example, a portion of a transposon end sequence of at least 10 bp (preferably at least 11 bp, more preferably at least 12 bp) in base length in which the 3' end side in the base sequence of a transposon end sequence known to be served as a non-transferred strand is deleted can be suitably used in the present invention.

For example, in the case of a Tn5-type transposase, a sequence such as 5'-AGATGTGTATAAGAGACAG-3' (SEQ ID NO: 1) is used as a transposon end sequence that serves as a transferred strand, and its complement, i.e., a sequence such as 5'-CTGTCTCTTATACACATCT-3' (SEQ ID NO: 2), is used as a transposon end sequence that serves as a non-transferred strand.

As shown in PTL 2, the transposon end sequence used when a transposase shows transposition activity to double-stranded DNA is not limited to the example shown here, and any transposon end sequence can be used in the present invention as long as the transposase used shows transposition activity.

In an embodiment, in the first adapter, the first tagment sequence and a sequence complementary to the additional oligo are added to the 5' end of the first transposon end sequence. For example, as shown in Fig. 2, 5'-TCGTCGGCAGCGTCCTCGTGTGCTACTTGAAGATGTGTATAAGAGACAG-3' (SEQ ID NO: 3) can be used as the first adapter that serves as a transferred strand, 5'-CTGTCTCTTATACACATCT-3' (SEQ ID NO: 4) can be used as the second adapter that serves as a non-transferred strand, and 5'-TCAAGTAGCACACGAGCCGAGCCCACGAGAC-3' (SEQ ID NO: 5) can be used as the additional oligo. The 5' ends of the second adapter and the additional oligo may be modified by phosphorylation. In addition, the OH group on the 3' end side of the additional oligo may be modified (e.g., modified with a phosphate group or inverted dT).

In another embodiment, the non-transferred strand can be shorter. For example, the total length of the non-transferred strand (or the second adapter that can be a non-transferred strand) can be shortened to about 10 bp to 19 bp, preferably about 11 bp to 19 bp, more preferably about 12 bp to 19 bp, and even more preferably about 14 bp to 19 bp.

For example, as shown in Fig. 3, 5 TCGTCGGCAGCGTCAGCTAGCTAGCAGATGTGTATAAGAGACAG-3' (SEQ ID NO: 6) can be used as the first adapter that serves as a transferred strand, 5'-CTGTCTCTTATACAC-3' (SEQ ID NO: 7) can be used as the second adapter that serves as a non-transferred strand, and 5'-ATCTGCTAGCTAGCTCCGAGCCCACGAGAC-3' (SEQ ID NO: 8) can be used as the additional oligo. The 5' ends of the second adapter and the additional oligo may be modified by phosphorylation. In addition, the OH group on the 3' end side of the additional oligo may be modified (e.g., modified with a phosphate group or inverted dT).

Thus, even if the adapter that is a non-transferred strand is short, it can form a stable tagment sequence together with the additional oligo and can be added to double-stranded DNA together with the adapter that is a transferred strand.

In another embodiment, in the first adapter, the first tagment sequence may be incorporated into a sequence complementary to the additional oligo. In other words, sequences complementary to the additional oligo may be present on the 5' end and 3' end sides of the first tagment sequence.

For example, as shown in Fig. 4, 5 CTCGTGTGCTACTTGATCGTCGGCAGCGTCAGATGTGTATAAGAGACAG-3' (SEQ ID NO: 9) can be used as the first adapter that serves as a transferred strand, 5'-CTGTCTCTTATA-3' (SEQ ID NO: 10) can be used as the second adapter that serves as a non-transferred strand, and 5'-CACATCTCCGAGCCCACGAGACTCAAGTAGCACACGAG-3' (SEQ ID NO: 11) can be used as the additional oligo. The 5' ends of the second adapter and the additional oligo may be modified by phosphorylation. In addition, the OH group on the 3' end side of the additional oligo may be modified (e.g., modified with a phosphate group or inverted dT).

When such a first adapter, second adapter, and additional oligo are used, a cyclic structure in which the first tagment sequence and the second tagment sequence are sandwiched between the double-stranded regions is formed. In the present invention, such tagment sequences may be added to double-stranded DNA. Forming such a cyclic structure allows the first and second tagment sequences to be present closer to the ends of double-stranded DNA; thus, there is an advantage in that, for example, the reaction is allowed to proceed more efficiently when PCR or the like is performed from the first and second tagment sequences using primers corresponding to the first and second tagment sequences.

### 5. Preparation of Transposome

The transposome used in the present invention is characterized by comprising at least the first adapter (a), the second adapter (b), and the transposase (c). The method for providing the first and second adapters to the transposase is not particularly limited, and examples include methods described in, for example, NPL 1 and Picelli et al., Genome Res. 24(12): 2033-40, 2014.

### 6. Reaction Temperature and Time for Transposase Treatment ((i) Step of Reacting Transposome and Double-stranded DNA)

The temperature at which the transposome and double-stranded DNA are reacted can be appropriately set according to, for example, the transposase used, the sequence and length of the first adapter, the sequence and length of the second adapter, or a combination of these. For example, the reaction temperature is preferably 30°C or more and 65°C or less, more preferably 37°C or more and 60°C or less, and even more preferably 50°C or more and 60°C or less. The reaction time is preferably 1 minute or more, more preferably 3 minutes or more, and even more preferably 5 minutes or more. The upper limit of the reaction time is not particularly limited, and is, for example, 24 hours or less, preferably 12 hours or less, more preferably 6 hours or less, and even more preferably 3 hours or less.

The concentration of the transposase in a reaction liquid in which the transposome and double-stranded DNA are reacted is not particularly limited as long as transposase activity is exhibited, and is preferably 10 pg/µL or more. The reaction liquid used for the transposase treatment is not particularly limited as long as transposase activity is exhibited, and may contain other additives. For example, the addition of N,N-dimethylformamide or polyethylene glycol is known to increase the efficiency of library preparation for NGS using transposon reaction, and these additives can be suitably used (Picelli et al., Genome Res. 24(12): 2033-40, 2014). In addition to these, for example, buffers, salts, organic solvents, polymers, surfactants, chelating agents, proteins, or a combination of two or more of these can be used as additives.

Examples of buffers include Tris, Bis-Tris, Tricine, Bis-Tricine, Hepes, Mops, Tes, Taps, Pipes, Caps, phosphoric acid, acetic acid, and a combination of two or more of these. Buffers are generally dissolved in water (preferably nuclease-free water) and used in the form of an aqueous solution.

Examples of salts include chlorides (e.g., lithium chloride, sodium chloride, potassium chloride, magnesium chloride, manganese chloride, and ammonium chloride), acetates (e.g., lithium acetate, sodium acetate, potassium acetate, magnesium acetate, manganese acetate, and ammonium acetate), sulfates (e.g., potassium sulfate, magnesium sulfate, manganese sulfate, and ammonium sulfate), and a combination of two or more of these.

Examples of organic solvents include alcohols (e.g., ethanol, methanol, and polyethylene glycol), aprotic solvents, such as N,N-dimethylformamide and dimethyl sulfoxide, and a combination of two or more of these.

Examples of surfactants include, but are not limited to, anionic surfactants (e.g., sodium dodecyl sulfate, sodium cholate, and sodium deoxycholate), cationic surfactants (e.g., cetyltrimethylammonium bromide), nonionic surfactants (e.g., octylphenol ethoxylate, polyoxyethylene lauryl ether, polyoxyethylene oleyl ether, polyoxyethylene stearyl ether, and polyoxyethylene sorbitan monolaurate), zwitterionic surfactants (e.g., 3-[(3-cholamidopropyl)dimethylammonio]-1-propanesulfonic acid), and the like.

Examples of chelating agents include, but are not limited to, ethylenediaminetetraacetic acid (EDTA), glycol ether diamine tetraacetic acid (EGTA), nitrilotriacetic acid (NTA), phenanthroline, maleic acid, citric acid, tricine, trans-1,2-diaminocyclohexane-N,N,N',N'-tetraacetic acid (CDTA), tartaric acid, nicotinamide, hydroxyethylimino diacetic acid (HIDA), phytic acid, and the like.

Examples of proteins include, but are not limited to, bovine serum albumin (BSA) and the like. Addition of a protein such as BSA can stabilize an enzyme and suppress the adsorption of reaction components to a container.

### 7. (ii) Linking of Additional Oligo to Second Adapter

The method for linking the additional oligo to the second adapter is not particularly limited, and is preferably performed by enzymatic reaction, more preferably using a polynucleotide ligase, particularly preferably using a DNA ligase. When a polynucleotide ligase is used, the origin of the enzyme is not particularly limited, and it is preferable to use a DNA ligase, such as DNA ligase derived from T4 phage, DNA ligase derived from T3 phage, DNA ligase derived from T7 phage, DNA ligase derived from *E. coli,* or a mutant thereof, or Ampligase (registered trademark) Thermostable DNA ligase sold by Lucigen.

When the additional oligo and the second adapter are one or more bases apart, the gap can be filled by enzymatic reaction of a DNA polymerase or the like (gap repair). This gap repair may be performed simultaneously with or separately from linking using a polynucleotide ligase. There is no particular limitation on enzymes used for gap repair and linking, and it is preferable to use a DNA polymerase and a polynucleotide ligase. The DNA polymerase is not particularly limited; it is preferable to use DNA polymerase derived from T4 phage, DNA polymerase I derived from *E. coli,* or Klenow fragment, which is a fragment of DNA polymerase I derived from *E. coli,* and it is more preferable to use an enzyme that shows almost no DNA strand displacement activity, such as DNA polymerase derived from T4 phage. The polynucleotide ligase may be of any origin, and it is preferable to use a thermostable DNA ligase, such as Ampligase (registered trademark) Thermostable DNA ligase sold by Lucigen, DNA ligase derived from T4 phage, or DNA ligase derived from *E. coli.*

When the linking of the additional oligo to the second adapter is performed by enzymatic reaction using enzymes described above, the concentrations of the enzymes are not particularly limited as long as they show activity, and it is preferable that the concentration of the polynucleotide ligase is 0.01 U/µL or more and that the concentration of the DNA polymerase is 0.005 U/µL or more. The reaction liquid used for the linking treatment in the enzymatic reaction is not particularly limited as long as it is a reaction liquid in which enzyme activity is exhibited, and may contain other additives. For example, it is known that polynucleotide ligases such as DNA ligase derived from T4 phage require adenosine triphosphate (ATP) for exhibiting their activity and that polynucleotide ligases such as DNA ligase derived from *E. coli* require nicotinamide adenine dinucleotide (NAD) for exhibiting their activity. DNA polymerases generally require deoxyribonucleotides for exhibiting their activity. Other additives, such as buffers, salts, organic solvents, polymers, surfactants, chelating agents, proteins, or a combination of two or more of these, may also be used.

Examples of buffers include Tris, Bis-Tris, Tricine, Bis-Tricine, Hepes, Mops, Tes, Taps, Pipes, Caps, phosphoric acid, acetic acid, and a combination of two or more of these. Buffers are generally dissolved in water (preferably nuclease-free water) and used in the form of an aqueous solution.

Examples of salts include chlorides (e.g., lithium chloride, sodium chloride, potassium chloride, magnesium chloride, manganese chloride, and ammonium chloride), acetates (e.g., lithium acetate, sodium acetate, potassium acetate, magnesium acetate, manganese acetate, and ammonium acetate), sulfates (e.g., potassium sulfate, magnesium sulfate, manganese sulfate, and ammonium sulfate), and a combination of two or more of these.

Examples of organic solvents include alcohols (e.g., ethanol, methanol, and polyethylene glycol), aprotic solvents, such as N,N-dimethylformamide and dimethyl sulfoxide, and a combination of two or more of these.

Examples of surfactants include, but are not limited to, anionic surfactants (e.g., sodium dodecyl sulfate, sodium cholate, and sodium deoxycholate), cationic surfactants (e.g., cetyltrimethylammonium bromide), nonionic surfactants (e.g., octylphenol ethoxylate, polyoxyethylene lauryl ether, polyoxyethylene oleyl ether, polyoxyethylene stearyl ether, and polyoxyethylene sorbitan monolaurate), zwitterionic surfactants (e.g., 3-[(3-cholamidopropyl)dimethylammonio]-1-propanesulfonic acid), and the like.

Examples of chelating agents include, but are not limited to, ethylenediaminetetraacetic acid (EDTA), glycol ether diamine tetraacetic acid (EGTA), nitrilotriacetic acid (NTA), phenanthroline, maleic acid, citric acid, tricine, trans-1,2-diaminocyclohexane-N,N,N',N'-tetraacetic acid (CDTA), tartaric acid, nicotinamide, hydroxyethylimino diacetic acid (HIDA), phytic acid, and the like.

Examples of proteins include, but are not limited to, bovine serum albumin (BSA) and the like. Addition of a protein, such as BSA, can stabilize an enzyme and suppress the adsorption of reaction components to a container.

The enzymes described above may be added to the reaction liquid used in step (i) (transposase treatment) to perform step (ii) (the linking of the additional oligo to the second adapter).

### 8. (iii) Linking of Adapter That Is Non-transferred Strand to Fragmented Double-stranded DNA

The method for linking the non-transferred strand (e.g., the second adapter) to fragmented double-stranded DNA is not particularly limited and is preferably performed by enzymatic reaction. When the adapter that is a non-transferred strand is linked to fragmented double-stranded DNA by enzymatic reaction, it is preferable to phosphorylate the 5' end of the non-transferred strand. In some embodiments, the non-transferred strand and fragmented double-stranded DNA may be several bases apart in treatment using the transposase, and so it preferable to perform gap repair.

The gap repair between the adapter that is a non-transferred strand and fragmented double-stranded DNA may be performed simultaneously with or separately from the linking of the additional oligo to the second adapter in (ii) described above. The enzyme used for the linking of the additional oligo to the second adapter in (ii) and the enzyme used for the linking of the adapter that is a non-transferred strand to fragmented double-stranded DNA in (iii) may be the same or different enzymes. There is no particular limitation on enzymes used for gap repair and linking, and it is preferable to use a DNA polymerase and a polynucleotide ligase. The DNA polymerase is not particularly limited; it is more preferable to use DNA polymerase derived from T4 phage, DNA polymerase I derived from *E. coli,* or Klenow fragment, which is a fragment of DNA polymerase I derived from *E. coli,* and it is more preferable to use an enzyme that shows almost no DNA strand displacement activity, such as DNA polymerase derived from T4 phage. The polynucleotide ligase may be of any origin, and it is preferable to use a thermostable DNA ligase, such as Ampligase (registered trademark) Thermostable DNA ligase sold by Lucigen, DNA ligase derived from T4 phage, or DNA ligase derived from *E. coli.*

When the adapter that is a non-transferred strand is linked to fragmented double-stranded DNA by enzymatic reaction using enzymes described above, the concentrations of the enzymes are not particularly limited as long as they show activity, and it is preferable that the concentration of the polynucleotide ligase is 0.01 U/µL or more and that the concentration of the DNA polymerase is 0.005 U/µL or more. The reaction liquid used for the linking treatment in the enzymatic reaction is not particularly limited as long as it is a reaction liquid in which enzyme activity is exhibited, and may contain other additives. For example, as the other additives, the same additives as those described above as other additives that can be suitably used in enzymatic reaction in the linking of the additional oligo to the second adapter in (ii) can be used.

### 9. Reaction Temperature and Time for Linking in (ii) and (iii)

The reaction conditions for step (ii) of linking the additional oligo to the second adapter and step (iii) of linking the adapter that is a non-transferred strand to fragmented double-stranded DNA are not particularly limited, and the reaction conditions for both steps may be the same or different. For example, when both steps are performed under the same reaction conditions, the upper limit of the reaction temperature is not particularly limited, and as an example, the upper limit of the reaction temperature is preferably less than or equal to the Tm value of the complementary sequence portion between the first adapter and the second adapter (in particular, the complementary sequence portion between the first transposon end sequence and the second transposon end sequence) + 15°C, and less than or equal to the Tm value of the complementary sequence portion between the first adapter and the additional oligo (in particular, the complementary sequence portion between a region other than the first tagment sequence (preferably the region of the optional sequence of the first adapter and/or the region of a portion of the first transposon end sequence)) and the additional oligo (a region other than the second tagment sequence)) + 15°C, and more preferably less than or equal to the Tm value of the complementary sequence portion between the first adapter and the second adapter + 10°C, and less than or equal to the Tm value of the complementary sequence portion between the first adapter and the additional oligo + 10°C. In an embodiment, the upper limit of the reaction temperature is preferably 45°C or less, and more preferably 37°C or less. In this case, the lower limit of the reaction temperature is not particularly limited, and is preferably 4°C or more, more preferably 10°C or more, and even more preferably 15°C or more, to reduce non-specific linking while maintaining enzyme activity. When linking is performed by enzymatic reaction, the temperature is preferably in a range in which polynucleotide ligase activity can be maintained at 10% or more and DNA polymerase activity can be maintained at 10% or more, to avoid a decrease in enzyme activity due to low temperature or heat.

The reaction time is not particularly limited, and is, for example, preferably 5 minutes or more, and more preferably 10 minutes or more. The upper limit of the reaction time is not particularly limited, and may be, for example, 1 hour or less.

In the method for generating a double-stranded DNA library of the present invention, DNA may or may not be purified after any one or more of steps (i), (ii), and (iii) described above or after all of these steps. When the PCR reaction described below is performed, DNA may or may not be purified before the PCR. Furthermore, when NGS analysis is performed using a double-stranded DNA library generated by the present invention, DNA may or may not be purified before the NGS analysis. DNA can be purified by any method known in the art. Examples include, but are not limited to, a method using AMPure XP (Beckman Coulter) or similar adsorbent beads and a method using insolubilization with ethanol or polyethylene glycol.

### 10. Polymerase Chain Reaction (PCR)

After addition of the tagment sequences in the present invention (after the completion of steps (i), (ii), and (iii)), the tagmented double-stranded DNA can be used for various applications. In the present invention, impurities, such as adapter dimers, are not generated, unlike the case of adding a Y-adapter; thus, the tagmented double-stranded DNA can be directly used for various applications without purification. For example, the tagmented double-stranded DNA may be amplified by any nucleic acid amplification method, such as PCR, using a primer pair designed to bind to the different tagment sequences added to the 5' end and 3' end sides. Moreover, new tagment sequences may be added by nucleic acid amplification methods, such as PCR. Examples of tagment sequences that can be added by nucleic acid amplification methods, such as PCR, include, but are not limited to, index sequences used in NGS instruments (e.g., NextSeq^{™} and MiSeq^{™} series) of Illumina, Inc., or the like, sequences for hybridization on a flow cell (e.g., p5 and p7 sequences of Illumina, Inc.), sequences required for recognition of target DNA in long-read sequencers (e.g., MinION, GridION, PromethION) of Oxford Nanopore Technologies, and the like. There is no particular limitation on the nucleic acid amplification methods, such as PCR.

### 11. Retention of Strand Information

In the present invention, strand information may or may not be retained in the library prepared. The target double-stranded DNA when strand information is retained is not particularly limited. For example, after synthesizing cDNA from RNA, DNA complementary to the cDNA is synthesized in a reaction liquid containing dUTP to prepare double-stranded DNA containing uracil in only one strand; in a later step, for example, treatment using UNG (uracil-DNA glycosylase) is performed and/or PCR is performed using a DNA polymerase that cannot use uracil containing DNA strand as a template (e.g., a family B DNA polymerase), so that the DNA strand containing uracil is rendered unamplifiable. This allows, for example, subsequent sequencing analysis of each DNA strand while retaining strand information.

### 12. Bisulfite Sequencing Analysis

According to the present invention, bisulfite sequencing analysis, especially tagmentation-based whole-genome bisulfite sequencing (T-WGBS) analysis, can also be easily performed. For example, a cytosine base in the first tagment sequence region of the first adapter and a cytosine base in the second tagment sequence of the additional oligo are methylated, a library is generated from double-stranded DNA, and treatment is performed using bisulfite or the like, thereby enabling detection of the methylation status of cytosines in the double-stranded DNA. Such bisulfite sequencing analysis can be performed, for example, by referring to the method described in Adey A. et al., Genome Res. 22, 1139-1143, 2012, or the like.

Thus, in one embodiment, the present invention may be a method for generating a double-stranded DNA library for use in bisulfite analysis, using the first adapter in which a cytosine base in the first tagment sequence is methylated and the additional oligo in which a cytosine bases in the second tagment sequence is methylated. This method allows methylation analysis of double-stranded DNA in which at least one strand is modified by methylation.

### 13. Double-stranded DNA

The double-stranded DNA targeted in the present invention (also referred to as "target double-stranded DNA") is not particularly limited, and examples include genomic DNA extracted from cells, double-stranded DNA amplified by a method such as PCR, double-stranded DNA synthesized from RNA, and the like. The double-stranded DNA may be double-stranded DNA derived from a single cell, tissue, organ, or organism (e.g., an animal, a plant, a fungus, a bacterium, or a virus) or may be double-stranded DNA derived from multiple cells, tissues, organs, or organisms. The amount of double-stranded DNA used in the present invention is not particularly limited.

### 14. Double-stranded DNA Library Generation Kit

An embodiment of the present invention can be a kit for generating a library of double-stranded DNA having different tagment sequences on the 5' end and 3' end sides. The kit comprises at least a transposome comprising (a) a first adapter containing a first tagment sequence and a first transposon end sequence, (b) a second adapter containing a second transposon end sequence having a base sequence complementary to at least a portion of the first transposon end sequence, and (c) a transposase. The kit also preferably comprises an enzyme such as a polynucleotide ligase or a DNA polymerase. These components (a) to (c), enzymes, and other additives that may be optionally contained may be in the form of a mixture in which the components are optionally mixed or in the form of a kit comprising each component separately.

### Examples

The present invention is described below in more detail with reference to Examples. However, the present invention is not limited to these Examples.

### Preparation of Transposome

Before the Examples, in order to prepare a transposase, a gene encoding a protein in which the C-terminus of hyperactive Tn5 (Tn5 transposase mutant (E54K/L372P mutant) described in Picelli et al., Genome Res. 24(12): 2033-40, 2014) was modified with a histidine tag was inserted into an expression plasmid and transformed into *E. coli* strain DH5α. The *E. coli* strain was cultured at 30°C, suspended in 50 mM HEPES-KOH (pH of 8.0) and 100 mM NaCl, and disrupted by sonication. The disruption liquid was subjected to affinity chromatography using TALON (trademark) Affinity Resin (ClonTech). After binding, washing was performed with 50 mM HEPES-KOH (pH of 8.0), 100 mM NaCl, and 10 mM imidazole, and elution was performed with 50 mM HEPES-KOH (pH of 8.0), 100 mM NaCl, and 150 mM imidazole, thereby obtaining purified hyperactive Tn5. The purified hyperactive Tn5 was dialyzed in 50 mM HEPES-KOH (pH of 7.5), 100 mM NaCl, 1 mM dithiothreitol (DTT), and 50% glycerol, buffer exchange was performed, and dilution was performed with a dialysis buffer so that the protein concentration was 100 µg/mL, to prepare a solution as a purified Tn5 solution. To prepare adapters to be provided for the transposase, the three sets of adapters shown in Table 1 were prepared.

**Table 1**

| A dapter Sets Used in Example 1 | |
|---|---|
| Adapter set | First adapter sequence (transferred strand) (5' -3' ) |
| | Second adapter sequence (non-transferred strand) (5' -3' ) |
| 1 | TCGTCGGCAGCGTCCTCGTGTGCTACTTGAAGATGTGTATAAGAGACAG |
| | (5'phosphorylation) CTGTCTCTTATACACATCT |
| 2 | TCGTCGGCAGCGTCAGCTAGCTAGCAGATGTGTATAAGAGACAG |
| | (5'phosphorylation) CTGTCTCTTATACAC |
| 3 | CTCGTGTGCTACTTGATCGTCGGCAGCGTCAGATGTGTATAAGAGACAG |
| | (5'phosphorylation) CTGTCTCTTATA |

Solutions were prepared so that the concentrations of the first adapter and the second adapter in each adapter set were 5 µM in a solution of 50 mM HEPES-KOH (pH of 7.5) and 100 mM NaCl, and heated to 100°C. Adapter annealing was then performed by cooling to 25°C at Δ-1°C/min. After annealing, an equal amount of 100% glycerol was mixed to prepare adapter solutions (1, 2, and 3) adjusted to 50% glycerol.

6 µL of any one of the adapter solutions (1, 2, and 3) was added to 24 µL of the purified Tn5 solution, and the mixture was allowed to stand at 25°C for 60 minutes to provide the adapters and diluted 100-fold with a dialysis buffer to prepare Tn5 (adapters 1), Tn5 (adapters 2), and Tn5 (adapters 3).

As negative controls, the same procedure was performed using the three adapter sets shown in Table 2 below, in which the 5' side of the second adapter (non-transferred strand) is not phosphorylated and which cannot bind to the target double-stranded DNA in an additional reaction using a DNA ligase or the like, to prepare Tn5 (adapters 1 NC), Tn5 (adapters 2 NC), and Tn5 (adapters 3NC).

Using the prepared Tn5 (adapters 1), Tn5 (adapters 2), Tn5 (adapters 3), Tn5 (adapters 1 NC), Tn5 (adapters 2 NC), and Tn5 (adapters 3 NC), activity was measured by evaluating degradation of human genomic DNA, and it was confirmed that all of them showed activity. The presence or absence of 5' phosphorylation of the adapters did not affect the transposition activity of the transposome.

**Table 2**

| Negative Controls Used in Example 1 | |
|---|---|
| Adapter set | First adapter sequence (transferred strand) (5' -3' ) |
| | Second adapter sequence (non-transferred strand) (5' -3' ) |
| 1 NC | TCGTCGGCAGCGTCCTCGTGTGCTACTTGAAGATGTGTATAGAGACAG |
| | CTGTCTCTTATACACATCT |
| 2NC | TCGTCGGCAGCGTCAGCTAGCTAGCAGATGTGTATAAGAGACAG |
| | CTGTCTCTTATACAC |
| 3 NC | CTCGTGTGCTACTTGATCGTCGGCAGCGTCAGATGTGTATAAGAGACAG |
| | CTGTCTCTTATA |

In Table 2, the solid underlines indicate first tagment sequences, and the dashed underlines indicate first transposon end sequences.

### Preparation of Additional Oligos

Further, the additional oligos shown in Table 3 to be used with the adapter sets were prepared at 10 µM.

**Table 3**

| Name of additional oligo | Sequence (5' -3' ) |
|---|---|
| Additional oligo 1 | (5'phosphorylation) TCAAGTAGCACACGAGCCGAGCCCAC GAAGAC (3' inverted dT modification) |
| Additional oligo 2 | (5'phosphorylation) ATCTGCTAGCTAGCTCCGAGCCCACGA GAC (3' inverted dT modification) |
| Additional oligo 3 | (5'phosphorylation)CACATCTCCGAGCCCACGAGACTCAAG TAGCACACGAG (3' inverted dT modification) |

In Table 3, the underlines indicate second tagment sequences.

### Preparation of Buffers for Reaction

As buffers for reaction, a buffer for Tn5 reaction (20 mM Tris-HCl (pH of 7.5), 15% dimethylformamide, 10 mM MgCl₂) and a buffer for additional reaction (300 mM Tris-HCl (pH of 8.0), 40 mM MgCl₂, 100 mM (NH₄)₂SO₄, 12 mM EDTA (pH of 8.0), 1 mM NAD, 0.05% BSA) were prepared.

### Example 1: Preparation of Library for NGS Using Present Invention

In this Example, the following experiment was conducted to examine whether the present invention enables efficient fragmentation of target double-stranded DNA and efficient tagmentation for adding different sequences to the 5' end and 3' end sides, making it possible to prepare a library for NGS.

Tn5 reaction liquids having the compositions shown in Table 4 were prepared using Tn5 (adapters) obtained by the preparation method described above and using genomic DNA (1 ng) purified from HeLa S3 cells as the target double-stranded DNA.

**Table 4**

| Tn5 Reaction Liquids | |
|---|---|
| 0.8 ng/µL HeLa S3 cell genomic DNA | 1.25 *µ*L |
| Tn5 (adapters 1, 2, or 3) or | 1.25 *µ*L |
| Tn5 (adapters 1 NC, 2 NC, or 3 NC) | |
| Buffer for Tn5 reaction | 2.5 *µ*L |
| Total | 5 *µ*L |

Each of the prepared reaction liquids was reacted at 55°C for 5 minutes (adapters 1, 2, 1 NC, and 2 NC) or at 50°C for 20 minutes (adapters 3 and 3 NC) for fragmentation and addition of the first tagment sequence. The reaction was stopped by adding 2.5 µL of a 0.15% sodium dodecyl sulfate solution, and each of additional reaction liquids having the compositions shown in Table 5 was individually added in a total amount.

**Table 5**

| Additional Reaction Liquids 1 | |
|---|---|
| 10 µM additional oligo 1, 2, or 3 | 0.5 *µ*L |
| Buffer for additional reaction | 1 *µ*L |
| 10 mM dNTPs (dATP, dTTP, dGTP, dCTP) | 0.5 *µ*L |
| 10 U/mL E. coli DNA ligase (NEB) | 0.05 *µ*L |
| 3 U/mL T4 DNA polymerase | 0.05 *µ*L |
| Sterilized water | 1.9 *µ*L |
| Total | 4 *µ*L |

Each of the additional reaction liquids was individually added and reacted at 37°C for 15 minutes (adapters 1, 2, 1 NC, and 2 NC) or at 20°C for 20 minutes (adapters 3 and 3 NC) for additional reaction to add the second tagment sequence. PCR reaction was performed using the thus obtained double-stranded DNA libraries without nucleic acid purification as follows.

A PCR reaction liquid having the composition shown in Table 6 was prepared, and PCR was performed using the reaction cycle shown in Table 7 to add index sequences and P5 and P7 sequences for Illumina NGS.

**Table 6**

| PCR Reaction Liquid | |
|---|---|
| Tagmented nucleic acid solution | 9 *µ*L |
| KOD-Plus-(1 U/µL) (Toyobo Co., Ltd.) | 0.6 *µ*L |
| 10× PCR Buffer for KOD -Plus- | 3 *µ*L |
| 2mM dNTPs | 3 *µ*L |
| 25 mM MgSO₄ | 1.8 *µ*L |
| N primer in Nextera XT Index Kit v2 (Illumina) | 3 *µ*L |
| S primer in Nextera XT Index Kit v2 (Illumina) | 3 *µ*L |
| Sterilized water | 6.6 *µ*L |
| Total | 30 *µ*L |

**Table 7**

| Reaction Cycle | | |
|---|---|---|
| Temperature | Time | |
| 94°C | 2 minutes | |
| 98°C | 10 seconds | 20 cycles |
| 60°C | 30 seconds | |
| 68°C | 30 seconds | |
| 4°C | h o l d * | |

| | | |
|---|---|---|
| * Within 1 hour at 4° C conditions | | |

After the reaction, the PCR products were subjected to nucleic acid purification using 24 µL of AMPure XP beads (Beckman Coulter) according to the manual, and elution was performed in 30 µL of TE buffer (Nacalai Tesque, Inc.) (purified PCR nucleic acid solutions). 3 µL of each purified PCR nucleic acid solution was subjected to electrophoresis using a Microchip Electrophoresis System for DNA/RNA Analysis MultiNA (Shimadzu Corporation) according to the manual to confirm the patterns of the library products (Fig. 5). In addition, the libraries were quantified using a GenNext (registered trademark) NGS Library Quantification Kit (produced by Toyobo Co., Ltd.) according to the manual, and the library yield was calculated to be 120 nM for Tn5 (adapters 1) and 0.4 nM for Tn5 (adapters 1 NC). Similar results were obtained for Tn5 (adapters 2) and Tn5 (adapters 2 NC), and Tn5 (adapters 3) and Tn5 (adapters 3 NC).

These results show that the present invention enables efficient fragmentation and tagmentation, making it possible to prepare libraries for Illumina NGS. Almost no yields were obtained using the negative controls, indicating that the reaction occurs when the additional oligo is linked to the second adapter that is a non-transferred strand, and the second adapter is linked to the target double-stranded DNA.

### Example 2: Preparation of Library for NGS in Which Strand Information Is Retained Using Present Invention

In this Example, the following experiment was conducted to examine whether the present invention enables efficient fragmentation of target double-stranded cDNA derived from RNA and efficient tagmentation, making it possible to prepare a library for NGS, and further whether strand information can be retained.

cDNA was synthesized from 1 ng of RNA purified from HeLa S3 cells using an RNeasy Plus Mini Kit (QIAGEN). The second-strand synthesis liquid shown in Table 8 was added to 9 µL of the cDNA solution, and reaction was performed using the cycle shown in Table 9 to obtain double-stranded DNA derived from RNA.

**Table 8**

| Second-strand Synthesis Liquid | |
|---|---|
| DNA Polymerase I, Large (Klenow) Fragment (NEB) | 0.25 *µ*L |
| 100 mM Tris-HCl, pH8.0 | 0.5 *µ*L |
| 500 mM NaCl | 0.5 *µ*L |
| 100 mM MgCl₂ | 0.5 *µ*L |
| 100 mM DTT | 0.05 *µ*L |
| 2 mM dNTPs (Toyobo Co., Ltd.) | 0.5 *µ*L |
| 2 mM dUTP (Toyobo Co., Ltd.) | 2 *µ*L or 0 *µ*L |
| 10 mM random primer, 6 mer | 0.5 *µ*L |
| Sterilized water | 1.2 *µ*L or 3.2 *µ*L |
| Total | 6 *µ*L |

**Table 9**

| Reaction Cycle | |
|---|---|
| Temperature | Time |
| 1 6 °C | 60 minutes |
| 7 0 °C | 10 minutes |
| 4 °C | h o l d * |

| | |
|---|---|
| *Within 1 hour at 4° C conditions | |

After the reaction, the double-stranded DNA derived from RNA was subjected to nucleic acid purification using 27 µL of AMPure XP beads (Beckman Coulter) according to the manual, and the DNA was eluted with a mixture of 2.5 µL of a buffer for Tn5 reaction and 1.25 µL of sterilized water (purified double-stranded DNA (derived from RNA)). Thereafter, as in Example 1, Tn5 reaction liquids having the compositions shown in Table 10 were prepared using the obtained purified double-stranded DNA (derived from RNA) as target double-stranded DNA.

**Table 10**

| Tn 5 Reaction Liquids | |
|---|---|
| Purified double-stranded DNA (derived from RNA) | 3.75 *µ*L |
| Tn5 (adapters 1, 2, or 3) | 1.25 *µ*L |
| Total | 5 *µ*L |

Each of the prepared reaction liquids was reacted at 55°C for 5 minutes (adapters 1 and 2) or at 50°C for 20 minutes (adapters 3) for fragmentation and addition of the first tagment sequence. The reaction was stopped by adding 2.5 µL of a 0.15% sodium dodecyl sulfate solution, and each of additional reaction liquids having the compositions shown in Table 5 was individually added in a total amount.

Each of the additional reaction liquids was individually added and reacted at 37°C for 15 minutes (adapters 1 and 2) or at 20°C for 20 minutes (adapters 3) for additional reaction to add the second tagment sequence. PCR reaction was performed using the thus obtained double-stranded DNA libraries without nucleic acid purification as follows.

A PCR reaction liquid having the composition shown in Table 6 was prepared, and PCR was performed using the reaction cycle shown in Table 7 to add index sequences and P5 and P7 sequences for Illumina NGS. In the PCR reaction liquid, a DNA polymerase derived from KOD (*Thermococcus kodakarensis*) belonging to family B, which cannot amplify base sequences containing uracil, was used.

After the reaction, the PCR products were subjected to nucleic acid purification using 24 µL of AMPure XP beads (Beckman Coulter) according to the manual, and elution was performed in 30 µL of TE buffer (Nacalai Tesque, Inc.) (purified PCR nucleic acid solutions). 3 µL of each purified PCR nucleic acid solution was subjected to electrophoresis using a Microchip Electrophoresis System for DNA/RNA Analysis MultiNA (Shimadzu Corporation) according to the manual to confirm the patterns of the library products (Fig. 6). In addition, the libraries were quantified using a GenNext (registered trademark) NGS Library Quantification Kit (Toyobo Co., Ltd.) according to the manual, and the library yield was calculated to be 83 nM in the condition without dUTP and 37 nM in the condition with dUTP for Tn5 (adapters 1). Similar results were obtained for Tn5 (adapters 2) and Tn5 (adapters 3).

These results show that the present invention enables efficient fragmentation and tagmentation even when cDNA derived from RNA is used, making it possible to prepare a library for Illumina NGS. Further, in the condition with dUTP, the yield was about half; thus, it was believed that the use of the polymerase that cannot perform amplification using uracil containing DNA strand as a template did not allow amplification from the second strand. This result shows that the double-stranded DNA libraries obtained by the method of this Example were composed of those amplified from the first strand and that strand information was retained.

### Example 3: NGS Analysis of Library Prepared Using Present Invention

Among the double-stranded DNA libraries obtained in Examples 1 and 2, the libraries prepared by treating genomic DNA or RNA-derived double-stranded DNA (with or without dUTP) with Tn5 (adapters 1), Tn5 (adapters 2), and Tn5 (adapters 3) were used to perform NGS analysis using a Miseq Reagent Kit v3 and Miseq. When adapters 1 and 2 were analyzed, the following custom primers were added as sequencing primers to the given sequencing primer liquid. For the analysis of adapters 3, the Read 1 primer, Read 2 primer, and Index 1 Read primer supplied with the Miseq Reagent Kit v3 were used.
Read 1 Primer for Adapters 1
   5'-GCGTCCTCGTGTGCTACTTGAAGATGTGTATAAGAGACAG-3' (SEQ ID NO: 12)
Read 2 Primer for Adapters 1
   5'-CTCGGCTCGTGTGCTACTTGAAGATGTGTATAAGAGACAG-3' (SEQ ID NO: 13)
Index 1 Read Primer for Adapters 1
   5'-TCAAGTAGCACACGAGCCGAGCCCACGAGAC-3' (SEQ ID NO: 14)
Read 1 Primer for Adapters 2
   5'-GGCAGCGTCAGCTAGCTAGCAGATGTGTATAAGAGACAG-3' (SEQ ID NO: 15)
Read 2 Primer for Adapters 2
   5'-GTGGGCTCGGAGCTAGCTAGCAGATGTGTATAAGAGACAG-3' (SEQ ID NO: 16)
Index 1 Read Primer for Adapters 2
   5'-ACATCTGCTAGCTAGCTCCGAGCCCACGAGAC-3' (SEQ ID NO: 17)

The CLC Genomics Workbench was used to analyze the mapping rate and the strand rate to the human genome for the analyzed NGS data. Table 11 shows the results.

**Table 11**

| Results of NGS Analysis | | |
|---|---|---|
| Sample | Mapping rate | Strand rate |
| Genomic DNA (adapters 1) | 9 9. 5% | - |
| Genomic DNA (adapters 2) | 9 8 . 5 % | - |
| Genomic DNA (adapters 3) | 9 9. 7% | - |
| NA-derived double-stranded DNA (adapters 1) (without UTP) | 9 2. 5% | - |
| RNA-derived double-stranded DNA (adapters 2) (without UTP) | 9 3. 2% | - |
| RNA-derived double-stranded DNA (adapters 3) (without UTP) | 9 2. 7% | - |
| RNA-derived double-stranded DNA (adapters 1) (with UTP) | 8 9. 7% | 9 1. 5% |
| RNA-derived double-stranded DNA (adapters 2) (with UTP) | 9 1. 8% | 9 5. 2% |
| RNA-derived double-stranded DNA (adapters 3) (with UTP) | 9 0 . 1% | 9 2. 2% |

These results reveal that the double-stranded DNA library generation method of the present invention can efficiently generate a double-stranded DNA library that can be used for sequence analysis or the like, including NGS, in a simple procedure.

### Industrial Applicability

The present invention enables simple and rapid preparation of libraries for gene analysis, such as next-generation sequencing (NGS) analysis.

## Claims

1. A method for generating a library of double-stranded DNA having different tagment sequences on the 5' end and 3' end sides, the method comprising the following steps (i) to (iii):
(i) fragmenting double-stranded DNA using a transposome comprising (a) a first adapter containing a first tagment sequence and a first transposon end sequence, (b) a second adapter containing a second transposon end sequence having a base sequence complementary to at least a portion of the first transposon end sequence, and (c) a transposase, and linking either the first adapter or the second adapter as a transferred strand to each of the 5' ends or 3' ends of the fragmented double-stranded DNA;
(ii) linking an additional oligo containing a second tagment sequence to the second adapter; and
(iii) linking the first adapter or the second adapter that is not linked to the fragmented double-stranded DNA, as a non-transferred strand, to each of the 5' ends or 3' ends of the fragmented double-stranded DNA to which the adapter as the transferred strand is not linked in step (i),
step (ii) being performed before, after, or simultaneously with step (iii).

2. The method according to claim 1, wherein the first adapter contains the first tagment sequence at a position closer to the 5' end than the first transposon end sequence, and serves as a transferred strand that is linked to each of the 5' ends of the fragmented double-stranded DNA using the transposase.

3. The method according to claim 1, wherein the additional oligo contains a base sequence complementary to a portion of the base sequence of the first adapter.

4. The method according to claim 1, wherein the transposase is hyperactive Tn5 transposase, and the first and second transposon end sequences are Tn5-type transposon end sequences.

5. The method according to claim 1, wherein in step (ii), the additional oligo is linked to the second adapter using a DNA ligase.

6. The method according to claim 1, wherein in step (iii), the adapter as the non-transferred strand that is not linked to the fragmented double-stranded DNA is linked to the fragmented double-stranded DNA using a DNA polymerase and a DNA ligase.

7. The method according to claim 1, wherein in step (iii), the adapter as the non-transferred strand that is not linked to the fragmented double-stranded DNA is the second adapter whose 5' end is modified by phosphorylation, and the second adapter is linked to each of the 3' ends of the fragmented double-stranded DNA.

8. The method according to claim 1, wherein in step (i), the first adapter is linked as the transferred strand to each of the 5' ends of the fragmented double-stranded DNA, and in step (ii), the additional oligo whose 5' end is modified by phosphorylation is linked to the 3' end of the second adapter.

9. The method according to claim 1, wherein in step (i), the first adapter is linked as the transferred strand to each of the 5' ends of the fragmented double-stranded DNA, and in step (ii), the additional oligo in which the OH group on the 3' end side is modified is linked to the 3' end of the second adapter.

10. The method according to claim 9, wherein the modification of the OH group is a modification with a phosphate group or inverted dT.

11. The method according to claim 1, wherein a double-stranded DNA library that retains strand information is generated.

12. The method according to claim 1, wherein at least one strand of the fragmented double-stranded DNA is modified.

13. The method according to claim 12, wherein the modification is a methylation modification.

14. The method according to claim 12, wherein a double-stranded DNA library for use in bisulfite analysis is generated using the first adapter in which a cytosine base in the first tagment sequence is methylated, and the additional oligo in which a cytosine base in the second tagment sequence is methylated.
